# FASCICULE DE BREVET EUROPEEN

(11) **EP 2 670 730 B1**
(45) Date de publication et mention de la délivrance du brevet: **27.06.2018**
(21) Numéro de dépôt: 12703289.4
(22) Date de dépôt: 03.02.2012
(51) Int. Cl.: C07C 209/48, C07C 253/30

(54) **PREPARATION DE DIAMINE VIA LA PREPARATION D'AMINONITRILE**
HERSTELLUNG VON DIAMIN DURCH HERSTELLUNG VON AMINONITRIL
PREPARATION OF DIAMINE VIA THE PREPARATION OF AMINONITRILE

(30) Priorité: 04.02.2011 FR 1150908
(43) Date de publication de la demande: 11.12.2013
(73) Titulaire: RHODIA OPERATIONS, 93306 Aubervilliers (FR)
(72) Inventeur: LECONTE, Philippe, F-68150 Ribeauville (FR)
(74) Mandataire: Benvenuti, Federica
(86) Numéro de dépôt international: PCT/EP2012/051882
(87) Numéro de publication internationale: WO 2012/104420

(56) Documents cités:
- DD-A- 58 306
- DD-A1- 222 011
- RO-A2- 69 448
- US-A- 4 172 091
- US-A1- 2008 293 973

## Description

La présente invention concerne un procédé continu de préparation de diamine via la préparation d'aminonitrile par réaction entre la monoamine correspondante et l'alcène nitrile correspondant.

La présente invention concerne plus particulièrement un procédé continu de préparation de diméthylaminopropylamine (N,N-dimethyl-1,3-propanediamine ou DMAPA) via la préparation de 3-(diméthylamino)propanenitrile (DMAPN) par réation de diméthylamine (DMA) avec l'acrylonitrile (AN).

La DMAPA possède de nombreuses applications notamment dans le secteur des agents de durcissement, des agents de liaison, des échangeurs d'ions, des agents d'aide à la flocculation, des pigments ou des pesticides. Les quantités de production de DMAPA vont donc en grandissant. Il est important, pour certaines de ces applications, de fournir un procédé de préparation de DMAPA pure, en continu à coût réduit.

Il est connu, notamment de US2008/0293973, des procédés de préparation de DMAPA en trois étapes : une première étape de réaction entre la DMA et l'AN pour former le diméthylaminopropionitrile (DMAPN) ; une seconde étape d'hydrogénation du DMAPN pour former la DMAPA ; et enfin une étape de purification de la DMAPA obtenue. Le plus souvent DMA et AN sont introduits, dans le réacteur, en quantité stoechiométrique ou avec un excès de DMA.
Cependant, l'inconvénient de l'introduction stoechiométrique est que le rendement n'est pas suffisant. La DMA a un point d'ébullition très faible (de l'ordre de 7°C), ainsi, lorsqu'elle est introduite en excès, si l'on souhaite recycler la DMA n'ayant pas réagi, une distillation doit être mise en oeuvre ainsi qu'une condensation des vapeurs de DMA obtenues, ce qui alourdit sensiblement les installations de production et augmente le coût de fabrication.
Il est par ailleurs déjà connu d'hydrogéner l'aminonitrile (DMAPN) obtenu en présence d'un hydroxyde de métal alcalin et d'un catalyseur (US 6 951 959).

Il est donc nécessaire de fournir un procédé de préparation de diamine et notamment de DMAPA, apportant une solution à tout ou partie des problèmes des procédés de l'état de la technique.

Un premier objectif de la présente invention est de fournir un procédé de préparation en continu de diamine, notamment de DMAPA, et permettant de recycler les réactifs introduits en excès.
Un objectif de l'invention est également de fournir un procédé avantageux d'un point de vue industriel.
Un autre objectif de la présente invention est de fournir un procédé de purification de la diamine obtenue, notamment DMAPA, qui permette d'obtenir une pureté supérieure à 99,5% et qui soit de mise en oeuvre aisée.
Un autre objectif encore de la présente invention est de fournir un procédé qui permette de:
- limiter la formation de sous-produits notamment de sous-produits difficiles voire impossible à séparer avec les techniques usuelles ;
- contrôler la chaleur de réaction ;
- éviter la dégradation des produits formés ;
- faciliter le recyclage de la monoamine introduite en excès ; et/ou
- augmenter la sélectivité et le rendement en diamine, notamment en DMAPA.
D'autres objectifs apparaîtront à la lecture de la description de l'invention.

La présente invention concerne un procédé continu (P) de préparation de diamine comprenant les étapes de :
(a) réaction entre l'alcène nitrile correspondant et la monoamine correspondante pour former l'aminonitrile correspondant, la monoamine étant introduite en excès molaire par rapport à l'alcène nitrile avec recyclage vers la réaction de la monoamine n'ayant pas réagi ; le recyclage de la monoamine met en oeuvre un système de séparation, qui permet de récupérer :
   - d'une part, l'aminonitrile dont une première fraction est envoyée vers un réacteur pour l'étape (b) et une deuxième fraction est envoyée vers un système d'absorption pour permettre la solubilisation de la monoamine en excès; et
   - d'autre part, la monoamine en excès sous forme gazeuse qui est envoyée vers ledit système d'absorption qui permet sa solubilisation dans ladite deuxième fraction d'aminonitrile.
(b) réduction de l'aminonitrile obtenu à l'étape (a) par l'hydrogène en présence d'au moins un hydroxyde de métal alcalin, d'eau et d'un catalyseur d'hydrogénation ;
(c) séparation de l'hydroxyde de métal alcalin par évaporation de la diamine obtenue à l'étape (b) et de l'eau ;
(d) distillation de la diamine,
   - l'alcène nitrile est choisi parmi les alcènes linéaires ou ramifiés, en C₂ à C₄, dans lesquels un atome d'hydrogène est remplacé par un groupe cyano conjugué et
   - la monoamine est une secondaire de formule générale R¹ R² NH, dans laquelle R¹ et R², identique ou différent, représente, un alkyl en C₁ à C₄.
Pour la présente invention « l'alcène nitrile correspondant » et la « monoamine correspondante » sont les composés permettant d'obtenir la diamine de structure souhaitée par mise en oeuvre du procédé de l'invention, en particulier par la mise en oeuvre de l'addition de Michael suivie d'une hydrogénation.

L'alcène nitrile comprend au moins une double liaison carbone-carbone. Il est choisi parmi les alcènes linéaires ou ramifiés, en C₂ à C₄, dans lesquels un atome d'hydrogène est remplacé par un groupe cyano conjugué, c'est-à-dire que l'alcène nitrile comprend une double liaison en position α,β par rapport au groupe cyano. Des exemples d'alcène en C₂ à C₄ sont notamment l'éthène, le propène, le 1-butène, le 2-butène, le 2-methylpropène. Des exemples de nitriles sont par exemple l'acrylonitrile, le but-2-enenitrile, le méthacrylonitrile, le pent-2-enenitrile, le 2-éthylacrylonitrile, le 2-methylbut-2-enenitrile et le 3-methylbut-2-ènenitrile.
De façon préférée, l'alcène nitrile est l'acrylonitrile (AN).

La monoamine est une amine secondaire de formule générale R¹R²NH, dans laquelle R¹ et R², identiques ou différents, représentent un alkyl en C₁ à C₄. A titre d'exemple d'alkyle en C₁ à C₄, on peut citer le méthyle, l'éthyle, le n-propyle, l'i-propyle, le 1-n-butyle, le 2-n-butyle, l'i-butyle, le t-butyle.
De préférence, la monoamine est la diméthylamine (DMA).
De préférence, l'aminonitrile intermédiaire est le 3-(diméthylamino)propanenitrile (DMAPN) résultant de l'addition de la diméthylamine (DMA) sur l'acrylonitrile (AN).
De façon particulièrement préférée, le procédé de l'invention concerne la préparation de DMAPA par hydrogénation de DMAPN
Lors de l'étape (a) du procédé de l'invention, la monoamine est introduite en excès, notamment un excès d'au moins 0,1% molaire par rapport à l'alcène nitrile, de préférence compris entre 1% et 50% molaire, de manière plus préférée compris entre 5% et 25% molaire.
De façon particulièrement avantageuse, l'étape (a) est réalisée dans un ou plusieurs réacteurs en série de type piston avec recyclage de la monoamine en excès et d'une partie de l'aminonitrile.
Il est possible de mettre en oeuvre deux réacteurs piston en série. Dans ce cas et de façon avantageuse le volume du deuxième réacteur peut être jusqu'à 2, 3, 4, 5, 10, 25 ou 50 fois plus grand que le volume du premier réacteur.
La monoamine, notamment la diméthylamine (DMA), a une température d'ébullition très faible (de l'ordre de 7°C pour la DMA) le recyclage de l'excès de monoamine se fait généralement par distillation puis condensation des vapeurs de monoamine. De manière habituelle, cette condensation nécessite une unité de réfrigération ou un compresseur ce qui induit un coût important.

Le procédé de la présente invention permet de résoudre ce problème en solubilisant dans une fraction de l'aminonitrile obtenu à l'étape (a) la monoamine en excès, recyclée, sortant du système de séparation tel que décrit dans la suite, sous forme gazeuse.
Le recyclage de la monoamine se fait au moyen d'un système d'absorption dans lequel la monoamine gazeuse est solubilisée dans une fraction de l'aminonitrile obtenu à l'étape (a). La monoamine peut être acheminée vers le système d'absorption par un dispositif de vide tel qu'une pompe à vide, par exemple un éjecteur , une pompe à anneau liquide, une pompe roots ou sèche, ou un assemblage de ces dispositifs. A titre d'exemple d'assemblages de dispositifs de vide, on peut citer, sans y être limité, l'assemblage pompe sèche puis éjecteur ou bien l'assemblage pompe roots puis pompe à anneau liquide.
De façon particulièrement avantageuse, le dispositif de vide permet également de réduire la pression dans la colonne de distillation et d'abaisser le point d'ébullition de l'aminonitrile obtenu limitant ainsi sa dégradation et par conséquent la formation de sous-produits indésirables.
A titre d'exemple de système d'absorption, on peut citer les colonnes d'absorption ou les réacteurs.
De façon avantageuse, la pression au niveau du dispositif de vide est comprise entre 0,01 et 0,08 MPa.
Le recyclage de l'étape (a) met en oeuvre un système de séparation, par exemple un flash et/ou une colonne de distillation, qui permet de récupérer :
- d'une part, l'aminonitrile dont une première fraction est envoyée vers un réacteur pour l'étape (b) et une deuxième fraction est envoyée vers un système d'absorption pour permettre la solubilisation de la monoamine en excès; et
- d'autre part, la monoamine en excès sous forme gazeuse qui est envoyée, par exemple au moyen d'un dispositif de vide, vers le système d'absorption qui permet sa solubilisation dans la deuxième fraction d'aminonitrile, le tout étant notamment ensuite recyclé vers la réaction de l'étape (a).
Ainsi et de façon avantageuse, une fraction de l'aminonitrile obtenu à l'étape (a), sert également de solvant à cette étape.
De manière générale, la monoamine et l'alcène nitrile peuvent être introduits directement dans le réacteur, une partie de la monoamine introduite est constituée de la monoamine recyclée solubilisée dans l'aminonitrile. L'introduction des réactifs dans le réacteur peut se faire par l'intermédiaire d'un mélangeur statique.

De manière préférée, la température lors de l'étape (a) est comprise entre 25 et 110°C. Lorsque plusieurs réacteurs sont utilisés lors de l'étape (a), la température dans chaque réacteur est indépendamment comprise entre 25 et 110°C.

Avantageusement, l'étape (b) est mise en oeuvre dans un réacteur comprenant des moyens de séparation du catalyseur tel qu'un décanteur et/ou un système de filtration tangentielle.

Le réacteur peut être un réacteur de type piston ou un réacteur agité, de préférence le réacteur est un réacteur de type piston.

L'appareillage convenant à la mise en oeuvre l'étape (b) du procédé de l'invention permet de réaliser un excellent contact gaz/liquide, une séparation rapide et efficace de ces deux phases après contact, une séparation continue de l'hydrogénat et du catalyseur ainsi que le recyclage de ce dernier, en un temps compatible avec une désactivation la plus faible possible du catalyseur.

Le réacteur de l'étape (b) est notamment un réacteur piston pouvant comprendre trois sections principales : une section de réaction piston fonctionnant selon le principe de la colonne à bulles avec circulation d'un lit de catalyseur en suspension, une section de séparation gaz/liquide et une section de séparation catalyseur/liquide avec recyclage du catalyseur et soutirage du liquide (hydrogénat). L'appareillage présente également, en sortie de réacteur piston, une zone de décantation des particules de catalyseur, la phase surnageante étant recyclée dans le réacteur piston par une première boucle externe comportant un prélèvement du milieu contenant la diamine, la phase décantée étant recyclée dans le réacteur piston par une seconde boucle externe.
Un tel appareillage est notamment décrit dans la demande WO2009/043906 (notamment p. 10, l. 6 à p. 12, l. 27).

Un autre appareillage pouvant être utilisé dans le procédé de l'invention est un appareillage mettant en oeuvre une filtration tangentielle. L'utilisation d'un tel appareillage consiste à filtrer de manière continue et tangentiellement sur un filtre à membrane au moins une partie d'un mélange réactionnel triphasique comportant une phase liquide dans laquelle se trouve notamment la diamine formée, une phase gazeuse comprenant de l'hydrogène et une phase solide catalytique. Cet appareillage permet de recycler le catalyseur tout en récupérant au moins une partie du filtrat contenant les produits de réaction. Un tel appareillage de filtration tangentielle est notamment décrit dans FR2749191. La mise en oeuvre d'un tel appareillage est décrite dans FR2834984.

Les catalyseurs convenant pour l'étape (b) sont les métaux de Raney tels que du nickel de Raney ou du cobalt de Raney. De façon préférée le catalyseur est du nickel de Raney, par exemple du nickel de Raney dopé.

Des éléments promotteurs, ou dopants, peuvent être avantageusement incorporés au métal de Raney ; ils sont alors choisis parmi les éléments appartenant aux groupes IIB, IVB, VIA, VIIB, VIII de la classification périodique des éléments. De façon préférée, les promotteurs sont choisis parmi le titane, le chrome, le zirconium, le vanadium, le molybdène, le manganèse, le cobalt, le nickel, le zinc, le fer et leurs associations en toutes proportions. La quantité en poids d'élément dopant dans le catalyseur est généralement inférieure à 10%, de préférence inférieure à 5%.

De façon avantageuse, le catalyseur peut être séparé, tout ou partie de ce catalyseur peut alors être soumise à une étape de régénération avant d'être recyclée vers l'étape (b).

Différents procédés de régénération peuvent être mis en oeuvre.
Un premier procédé de régénération comprend :
(i) une étape de lavage à l'eau du catalyseur (notamment afin d'éliminer la plus grande partie des composés organiques) ;
(ii) une étape de traitement par une base; et
(iii) une étape de lavage avec une solution aqueuse d'hydroxyde alcalin ou de l'eau.

L'étape (i) est avantageusement répétée pour obtenir dans la dernière eau de lavage une concentration en composés organiques inférieure ou égale à 1% en poids. Cette concentration peut notamment être déterminée par la quantité de diamine contenue dans l'eau de lavage. Cette étape peut également être réalisée en continu dans une ou plusieurs colonnes à flux à contre-courant ou dans une colonne comprenant le catalyseur à régénérer sous forme de lit-fixe. Avantageusement, la température lors de cette étape (i) est comprise entre 10 et 50°C, de préférence entre 20 et 35°C (température ambiante).

Les bases convenants pour l'étape (ii) sont notamment les hydroxydes de métaux alcalins, de préférence la soude. La solution de base utilisée contient de préférence de 10 à 25% en poids d'hydroxyde alcalin. Cette étape est avantageusement réalisée à une température supérieure à 80°C et de préférence supérieure à la température d'ébullition de la base.
La durée de cette étape (ii) doit être suffisante pour permettre une régénération de l'activité catalytique correspondant à au moins 60% de l'activité catalytique du catalyseur vierge, avantageusement au moins 65%. Ce traitement permet avantageusement de produire de l'hydrogène qui va conditionner le catalyseur pour son utilisation ultérieure.
Le lavage de l'étape (iii) peut être répété, il est avantageusement réalisé avec de l'eau à une température comprise entre 40 et 90°C. Il est également possible d'utiliser comme liquide de lavage, une solution aqueuse d'hydroxyde de métal alcalin, de préférence de la soude, à une concentration minimale de 0,012g/l d'hydroxyde de métal alcalin, avantageusement comprise entre 0,012% en poids et 0,040% en poids d'hydroxyde de métal alcalin. Le lavage est avantageusement réalisé jusqu'à l'obtention dans la dernière eau de lavage, d'une concentration pondérale en hydroxyde alcalin (soude) supérieure ou égale à 0,012%.

Ce premier procédé de régénération peut être mis en oeuvre sous atmosphère inerte ou ne comprenant pas d'oxygène. Ce procédé est notamment tel que décrit dans FR2921922.

Un deuxième procédé de régénération consiste en un traitement à l'hydrogène comprenant
(i) une étape de lavage à l'eau du catalyseur (notamment afin d'éliminer la plus grande partie des composés organiques) ;
(ii) une étape de traitement sous pression d'hydrogène dans une solution de soude à une température inférieure à 130°C ;
(iii) le lavage du catalyseur par de l'eau ou une solution aqueuse basique, notamment jusqu'à un pH final des eaux de lavage compris entre 12 et 13 ;
Un tel procédé est notamment décrit dans FR2773086.

Enfin, il est possible de régénérer le catalyseur par traitement à l'eau. Un tel procédé de régénération est notamment celui décrit dans US6951959 (col.5, l. 45-58) ou dans US4429159.

Ces différents procédés de régénération permettent d'obtenir un catalyseur régénéré présentant une activité catalytique correspondant à 35 à 100%, de préférence à 40 à 90% de l'activité catalytique du catalyseur vierge.

Par catalyseur vierge on entend un catalyseur n'ayant pas été utilisé dans la réaction d'hydrogénation du procédé de l'invention.

La définition et le calcul de l'activité d'un catalyseur sont tels que décrits aux pages 5 à 7 de la demande internationale WO2009/043906.

Après lavage, le catalyseur régénéré est recyclé vers la réaction de l'étape (b) ou bien mélangé avec du catalyseur vierge avant recyclage vers la réaction de l'étape (b), notamment avant introduction dans le réacteur de l'étape (b).
Ainsi, selon un mode de réalisation, il est possible de mettre en oeuvre un mélange de catalyseur vierge et de catalyseur régénéré.
Le rapport massique de catalyseur vierge par rapport au catalyseur régénéré est compris entre 1/99 et 80/20.
Selon un autre mode de réalisation l'étape (b) ne met en oeuvre que du catalyseur vierge.

L'étape (b) peut être réalisée en présence d'un solvant qui peut avantageusement être le produit de l'hydrogénation.

La quantité d'eau mise en oeuvre lors de l'étape (b) est inférieure ou égale à 50%, avantageusement inférieure ou égale à 20% en poids de la phase liquide du flux réactionnel total de l'étape (b), par exemple comprise entre 0,1% et 15% en poids.

De façon préférée, à l'étape (b) l'hydroxyde de métal alcalin est choisi parmi LiOH, NaOH, KOH, RbOH, CsOH et leurs mélanges. NaOH et/ou KOH sont particulièrement préférés. Dans un mode de réalisation particulier, un seul hydroxyde de métal alcalin est mis en oeuvre lors l'étape (b).
La quantité d'hydroxyde de métal alcalin mise en oeuvre est d'au moins 0,1 mole d'hydroxyde de métal alcalin par kilogramme de catalyseur, de préférence entre 0,1 et 2 moles, par exemple entre 0,3 et 1,5 mole.

De manière avantageuse, l'étape (b) peut être menée à une température inférieure ou égale à 150°C, de préférence inférieure ou égale à 120°C et encore plus préférentiellement inférieure ou égale à 100°C, par exemple entre 50 et 100°C.

La pression d'hydrogène lors de l'étape (b) est comprise entre environ 0,1 et environ 10 MPa, de préférence entre 1 et 5 MPa.

La mise en oeuvre de l'étape (b) dans les conditions de l'invention, notamment choix du catalyseur, permet de limiter la formation de sous-produits, notamment il se forme très peu, voir pas, de N,N,N',N'-tetraméthyl-1,3-propanediamine qui serait difficilement séparable de la diamine.

L'étape (c) concerne l'élimination de la fraction d'hydroxyde de métal alcalin introduit à l'étape (b) soluble dans le milieu réactionnel (diamine + eau) par évaporation de la diamine et de l'eau.
La fraction d'hydroxyde de métal alcalin soluble dans le milieu réactionnel peut notamment être éliminée à l'aide d'un évaporateur. Dans l'évaporateur, l'eau et la majeure partie de la diamine issues de l'étape (b) passent en phase vapeur et sont dirigées vers une colonne de distillation pour l'étape (d).
Dans un mode de réalisation, la fraction résiduelle de diamine associée à l'hydroxyde de métal alcalin sortant de l'évaporateur peut être récupérée par décantation après solubilisation de l'hydroxyde de métal alcalin dans l'eau.
Selon ce mode de réalisation, l'hydroxyde de métal alcalin et la fraction résiduelle de diamine sortent en pied de l'évaporateur. De façon avantageuse, une alimentation en eau permet de dissoudre l'hydroxyde de métal alcalin contenu dans ce pied de l'évaporateur. Enfin et de façon avantageuse, un décanteur peut être positionné en pied de l'évaporateur permettant de récupérer d'une part l'hydroxyde de métal alcalin soluble dans l'eau et d'autre part la diamine non soluble dans le mélange eau+hydroxyde de métal alcalin qui peut être redirigée vers la colonne de distillation pour l'étape (d).

La colonne de distillation de l'étape (d) peut être une colonne à soutirage latéral ou une colonne à paroi.
De manière avantageuse, l'utilisation d'une colonne à soutirage latérale ou d'une colonne à paroi permet d'améliorer la séparation des impuretés contenues dans la diamine obtenue, notamment les impuretés telles que le N-méthyl-1,3-diaminopropane méthylaminopropylamine.

La distillation peut être réalisée à pression atmosphérique ou sous un léger vide, par exemple à une pression comprise entre 80 et 95 kPa.

Lors de la distillation, l'eau et les sous produits légers sortent en tête de colonne. Le soutirage de la diamine se fait sur un soutirage intermédiaire entre la tête et le pied de la colonne et les sous produits lourds sortent en pied de colonne.

La diamine obtenue par le procédé de l'invention est caractérisé par une pureté supérieure à 99,5%, notamment supérieure à 99,8%.

La présente invention concerne également un dispositif pour la préparation d'une diamine, comprenant :
(a) au moins un premier réacteur pour la réaction entre la monoamine correspondante et l'alcène nitrile correspondant pour former l'aminonitrile correspondant, et un système de recyclage de la monoamine introduite en excès ;
(b) un deuxième réacteur possédant des moyens de séparation du catalyseur pour l'hydrogénation de l'aminonitrile obtenue, en présence d'un hydroxyde de métal alcalin, d'un catalyseur d'hydrogénation et d'eau ;
(c) un évaporateur permettant de séparer l'hydroxyde de métal alcalin soluble dans le mélange eau + diamine ;
(d) une colonne de distillation permettant la purification de la diamine.

Le premier réacteur peut être un réacteur piston. Il est possible d'avoir plusieurs réacteurs piston en série.

Le système de recyclage est constitué d'un système de séparation, notamment un flash et/ou une colonne de distillation et d'un dispositif de vide associé à un système d'absorption.
Le système de séparation permet de récupérer :
- d'une part l'aminonitrile dont une première fraction est envoyée vers le réacteur d'hydrogénation et une deuxième fraction est envoyée vers le système d'absorption pour permettre la solubilisation de la monoamine en excès; et
- d'autre part la monoamine en excès sous forme gazeuse qui est envoyée au moyen d'un dispositif de vide, vers le système d'absorption qui permet sa solubilisation dans la deuxième fraction d'aminonitrile, le tout étant suite envoyé vers le premier réacteur.
A titre d'exemple de dispositif de vide, on peut citer par exemple une pompe à anneau liquide, un éjecteur, une pompe roots ou sèche, ou un assemblage de ces dispositifs. A titre d'exemple d'assemblage de dispositif de vide on peut citer, sans y être limité, l'assemblage pompe sèche puis éjecteur ou bien l'assemblage pompe roots puis pompe à anneau liquide. Le système d'absorption peut être une colonne d'absorption ou un réacteur.
Le réacteur d'hydrogénation peut être un réacteur piston ou un réacteur agité.
Les moyens de séparation du catalyseur peuvent être un décanteur et/ou un dispositif de filtration tangentielle.

Dans un mode de réalisation, dans l'évaporateur, l'eau et la majeure partie de la diamine passent en phase vapeur et est redirigée vers une colonne de distillation pour purification de la diamine.
Dans un mode de réalisation, la fraction résiduelle de diamine associée à l'hydroxyde de métal alcalin sortant de l'évaporateur est récupérée par décantation après solubilisation de l'hydroxyde de métal alcalin dans l'eau.
Selon ce mode de réalisation, l'hydroxyde de métal alcalin et la fraction résiduelle de diamine sortent en pied de l'évaporateur. De façon avantageuse, une alimentation en eau au niveau du pied de l'évaporateur permet de dissoudre l'hydroxyde de métal alcalin. Enfin et de façon avantageuse, un décanteur peut être positionné en pied de colonne permettant de récupérer, d'une part l'hydroxyde de métal alcalin soluble dans l'eau et d'autre part, la diamine non soluble dans le mélange eau+hydroxyde de métal alcalin qui peut être dirigée vers la colonne de distillation pour purification de la diamine.

La colonne de distillation permettant la purification de la diamine est avantageusement une colonne à soutirage latéral ou une colonne à paroi.

Toutes les caractéristiques essentielles et préférées du procédé (P) s'appliquent au dispositif de la présente invention.

Les conversions obtenues pour chacune des étapes du procédé de l'invention et pour le procédé (P) dans sa globalité sont supérieures à 99%.
La sélectivité de chacune des étapes et du procédé (P) est quant à elle supérieure à 99%.

Les exemples suivants illustrent le procédé de l'invention.

### Exemple 1: Synthèse de DMAPN

680 kg/h d'AN, 578 kg/h de DMA et 1397 kg/h d'une solution de DMA à 8,2% dans le DMAPN sont introduits via un mélangeur statique dans un réacteur tubulaire maintenu à 65°C. Le mélange réactionnel sortant du réacteur alimente une colonne à distiller sous 0,05 MPa.

La DMA sortant en tête de colonne ainsi que la moitié du DMAPN sortant en pied sont recyclés vers une colonne d'absorption fonctionnant à 30°C. La production de DMAPN représente 1258 kg/h avec une pureté supérieure ou égale 99,6%.

### Exemple 2: Hydrogénation du DMAPN

Dans un réacteur agité à 80°C sous une pression de 2,5 MPa d'hydrogène, sont introduits 629 kg/h de DMAPN, 27 kg/h d'eau à 0,1% de NaOH et l'hydrogène pour maintenir la pression constante. Le réacteur contient 5% en poids de Nickel de Raney dopé à 1,6% en poids de Ti avec 1,2 mol de NaOH par kg de nickel et est équipé d'une boucle de circulation comprenant un filtre tangentiel au travers duquel est soutiré 681 kg/h de DMAPA brute.

### Exemple 3: Séparation de l'hydroxyde de métal alcalin et distillation de la DMAPA

1362 kg/h de DMAPA brute alimentent un évaporateur sous pression atmosphérique. Les composés sortant en tête de l'évaporateur sont envoyés, sous forme gazeuse, dans une colonne de distillation. Les composés sortant en pied à 127°C sont soutirés à raison de 7,8 kg/h et sont mélangés à 0,25 kg/h d'eau, un décanteur perment une séparation d'une part de la phase aqueuse de soude et d'autre part de la DMAPA qui est envoyée, sous forme liquide, vers la colonne de distillation. La colonne de distillation fonctionne sous 0,095 MPa avec une température de pied de 145°C.
- 56 kg/h d'eau sont récupérés en tête de colonne contenant environ 3 % d'amines légères.
- 1300 kg/h d'une fraction intermédiaire de DMAPA pure avec un titre supérieur ou égal à 99,8% est soutirée latéralement de la colonne à une température de 133°C.
- 6 kg/h constituent le pied de colonne.

## Revendications

1. Procédé continu (P) de préparation d'une diamine comprenant les étapes de :
(a) réaction entre l'alcène nitrile correspondant et la monoamine correspondante pour former l'aminonitrile correspondant, la monoamine étant introduite en excès molaire par rapport à l'alcène nitrile, avec recyclage vers la réaction de la monoamine n'ayant pas réagi; le recyclage de la monoamine met en oeuvre un système de séparation, qui permet de récupérer :
- d'une part, l'aminonitrile dont une première fraction est envoyée vers un réacteur pour l'étape (b) et une deuxième fraction est envoyée vers un système d'absorption pour permettre la solubilisation de la monoamine en excès; et
- d'autre part, la monoamine en excès sous forme gazeuse qui est envoyée vers ledit système d'absorption qui permet sa solubilisation dans ladite deuxième fraction d'aminonitrile.
(b) réduction de l'aminonitrile obtenu à l'étape (a) par l'hydrogène en présence d'au moins un hydroxyde de métal alcalin, d'eau et d'un catalyseur d'hydrogénation ;
(c) séparation de l'hydroxyde de métal alcalin par évaporation de la diamine obtenue à l'étape (b) et de l'eau ;
(d) distillation de la diamine ;
- l'alcène nitrile est choisi parmi les alcènes linéaires ou ramifiés, en C₂ à C₄, dans lesquels un atome d'hydrogène est remplacé par un groupe cyano conjugué et
- la monoamine est une secondaire de formule générale R¹R²NH, dans laquelle R¹ et R², identique ou différent, représente, un alkyl en C₁ à C₄.

2. Procédé selon la revendication 1, pour lequel la monoamine est la diméthylamine, l'alcène nitrile est l'acrylonitrile, l'aminonitrile est le 3-(diméthylamino)propanenitrile et la diamine est la diméthylaminopropylamine.

3. Procédé selon l'une quelconque des revendications 1 à 3, pour lequel l'étape (a) est réalisée dans un ou plusieurs réacteurs en série de type piston avec recyclage de la monoamine en excès et recyclage d'une partie de l'aminonitrile.

4. Procédé selon l'une quelconque des revendications 1 à 3, pour lequel lors de l'étape (a) la monoamine est introduite en excès d'au moins 0,1% molaire par rapport à l'alcène nitrile.

5. Procédé selon l'une quelconque des revendications 1 à 4, pour lequel l'étape (b) est mise en oeuvre dans un réacteur comprenant des moyens de séparation du catalyseur

6. Procédé selon l'une quelconque des revendications 1 à 5, pour lequel le catalyseur de l'étape (b) est séparé, tout ou partie de ce catalyseur est régénéré avant d'être recyclé dans la réaction (b).

7. Procédé selon l'une quelconque des revendications 1 à 6, pour lequel le catalyseur de l'étape (b) est un métal de Raney.

8. Procédé selon l'une quelconque des revendications 1 à 7, pour lequel
- la fraction d'hydroxyde de métal alcalin soluble dans le milieu réactionnel est éliminée à l'aide d'un évaporateur ;
- l'eau et la majeure partie de la diamine issues de l'étape (b), passant en phase vapeur sont dirigées vers une colonne de distillation pour l'étape (d).

9. Procédé selon la revendication 8, pour lequel la fraction résiduelle de diamine associée à l'hydroxyde de métal alcalin sortant de l'évaporateur est récupérée par décantation après solubilisation de l'hydroxyde de métal alcalin dans de l'eau.

10. Procédé selon l'une quelconque des revendications 1 à 9, pour lequel, lors de l'étape (c) la distillation est réalisée au moyen d'une colonne à soutirage latéral ou d'une colonne à paroi.

11. Dispositif pour la préparation de diamine, comprenant :
(a) au moins un premier réacteur pour la réaction entre la monoamine correspondante et l'alcène nitrile correspondant pour former l'aminonitrile correspondant, et un système de recyclage de la monoamine introduite en excès ;
(b) un deuxième réacteur possédant des moyens de séparation du catalyseur pour l'hydrogénation de l'aminonitrile obtenue, en présence d'un hydroxyde de métal alcalin, d'un catalyseur d'hydrogénation et d'eau ;
(c) un évaporateur permettant de séparer l'hydroxyde de métal alcalin soluble dans le mélange eau + diamine ;
(d) une colonne de distillation permettant la purification de la diamine ;
pour lequel le système de recyclage est constitué d'un système de séparation, d'un dispositif de vide et d'un système d'absorption ;
pour lequel le système de séparation permet de récupérer :
- d'une part l'aminonitrile dont une première fraction est envoyée vers le réacteur d'hydrogénation et une deuxième fraction est envoyée vers le système d'absorption pour permettre la solubilisation de la monoamine en excès; et
- d'autre part la monoamine en excès sous forme gazeuse qui est envoyée au moyen d'un dispositif de vide, vers le système d'absorption qui permet sa solubilisation dans la deuxième fraction d'aminonitrile le tout est suite envoyé vers le premier réacteur.

12. Dispositif selon la revendication 11, pour lequel la colonne de distillation permettant la purification de la diamine est une colonne à soutirage latéral ou une colonne à paroi.

## Patentansprüche

1. Kontinuierliches Verfahren (P) zur Herstellung eines Diamins, das folgende Schritte umfasst:
(a) Umsetzen des entsprechenden Alkennitrils und des entsprechenden Monoamins zu dem entsprechenden Aminonitril, wobei das Monoamin in molarem Überschuss, bezogen auf das Alkennitril, eingetragen wird, mit Rezyklierung des nicht umgesetzten Monoamins zur Umsetzung; wobei bei der Rezyklierung des Monoamins ein Trennsystem verwendet wird, das die Gewinnung
- einerseits des Aminonitrils, von dem eine erste Fraktion einem Reaktor für Schritt (b) zugeführt wird und eine zweite Fraktion einem Absorptionssystem zur Solubilisierung des überschüssigen Monoamins zugeführt wird; und
- andererseits des überschüssigen Monoamins in gasförmiger Form, das dem Absorptions system, das dessen Solubilisierung in der zweiten Aminonitril-Fraktion ermöglicht, zugeführt wird,
ermöglicht;
(b) Reduzieren des in Schritt (a) erhaltenen Aminonitrils mit Wasserstoff in Gegenwart von mindestens einem Alkalimetallhydroxid, Wasser und einem Hydrierkatalysator;
(c) Abtrennen des Alkalimetallhydroxids durch Verdampfen des in Schritt (b) erhaltenen Diamins und des Wassers;
(d) Destillieren des Diamins;
- wobei das Alkennitril aus linearen oder verzweigten C₂- bis C₄-Alkenen, in denen ein Wasserstoffatom durch eine konjugierte Cyanogruppe ersetzt ist, ausgewählt wird und
- es sich bei dem Monoamin um ein sekundäres Monoamin der allgemeinen Formel R¹R²NH handelt, in der R¹ und R² gleich oder verschieden sind und für ein C₁- bis C₄-Alkyl stehen.

2. Verfahren nach Anspruch 1, für das es sich bei dem Monoamin um Dimethylamin handelt, es sich bei dem Alkennitril um Acrylnitril handelt, es sich bei dem Aminonitril um 3-(Dimethylamino)propannitril handelt und es sich bei dem Diamin um Dimethylaminopropylamin handelt.

3. Verfahren nach einem der Ansprüche 1 bis 3, für das Schritt (a) in einem oder mehreren Reaktoren in Reihe vom Kolbentyp mit Rezyklierung des überschüssigen Monoamins und Rezyklierung eines Teils des Aminonitrils durchgeführt wird.

4. Verfahren nach einem der Ansprüche 1 bis 3, für das in Schritt (a) das Monoamin in einem Überschuss von mindestens 0,1 Mol-%, bezogen auf das Alkennitril, eingetragen wird.

5. Verfahren nach einem der Ansprüche 1 bis 4, für das Schritt (b) in einem Reaktor mit Mitteln zur Abtrennung des Katalysators durchgeführt wird.

6. Verfahren nach einem der Ansprüche 1 bis 5, für das der Katalysator von Schritt (b) abgetrennt wird und dieser Katalysator ganz oder teilweise regeneriert und dann zur Umsetzung (b) rezykliert wird.

7. Verfahren nach einem der Ansprüche 1 bis 6, für das es sich bei dem Katalysator von Schritt (b) um ein Raney-Metall handelt.

8. Verfahren nach einem der Ansprüche 1 bis 7, für das
- die in dem Reaktionsmedium lösliche Fraktion von Alkalimetallhydroxid mit Hilfe eines Verdampfers entfernt wird;
- das Wasser und der überwiegende Teil des Diamins aus Schritt (b) in die Dampfphase übergehen und einer Destillationskolonne für Schritt (d) zugeführt werden.

9. Verfahren nach Anspruch 8, für das die mit dem aus dem Verdampfer austretenden Alkalimetallhydroxid assoziierte Diamin-Restfraktion durch Dekantieren nach Solubilisierung des Alkalimetallhydroxids in Wasser gewonnen wird.

10. Verfahren nach einem der Ansprüche 1 bis 9, für das in Schritt (c) die Destillation mit Hilfe einer Kolonne mit Seitenabzug oder einer Wandkolonne durchgeführt wird.

11. Vorrichtung zur Herstellung von Diamin mit:
(a) mindestens einem ersten Reaktor für die Umsetzung des entsprechenden Monoamins und des entsprechenden Alkennitrils zu dem entsprechenden Aminonitril und ein System zur Rezyklierung des im Überschuss eingetragenen Monoamins;
(b) einem zweiten Reaktor mit Mitteln zur Abtrennung des Katalysators für die Hydrierung des erhaltenen Aminonitrils in Gegenwart von einem Alkalimetallhydroxid, einem Hydrierkatalysator und Wasser;
(c) einem Verdampfer, der die Abtrennung des löslichen Alkalimetallhydroxids in der Mischung von Wasser und Diamin ermöglicht;
(d) einer Destillationskolonne, die die Reinigung des Diamins ermöglicht;
für die das Rezyklierungssystem aus einem Trennsystem, einer Vakuumsvorrichtung und einem Absorptionssystem besteht;
für die das Trennsystem die Gewinnung
- einerseits des Aminonitrils, von dem eine erste Fraktion dem Hydrierreaktor zugeführt wird und eine zweite Fraktion dem Absorptionssystem zur Solubilisierung des überschüssigen Monoamins zugeführt wird; und
- andererseits des überschüssigen Monoamins in gasförmiger Form, das mit Hilfe einer Vakuum vorrichtung dem Absorptions system, das dessen Solubilisierung in der zweiten Aminonitril-Fraktion ermöglicht, zugeführt wird, wobei das Ganze dann dem ersten Reaktor zugeführt wird; l
ermöglicht.

12. Vorrichtung nach Anspruch 11, für die es sich bei der Destillationskolonne, die die Reinigung des Diamins ermöglicht, um eine Kolonne mit Seitenabzug oder eine Wandkolonne handelt.

## Claims

1. Continuous process (P) for preparing a diamine, comprising the steps of:
(a) reaction between the corresponding alkenenitrile and the corresponding monoamine to form the corresponding aminonitrile, the monoamine being introduced in molar excess relative to the alkenenitrile, with recycling into the reaction of the unreacted monoamine; the recycling of the monoamine uses a separation system which makes it possible to recover:
- on the one hand, the aminonitrile, a first fraction of which is sent to a reactor for step (b) and a second fraction of which is sent to an absorption system to enable dissolution of the excess monoamine; and
- on the other hand, the excess monoamine in gaseous form, which is sent to said absorption system which enables its dissolution in said second fraction of aminonitrile.
(b) reduction of the aminonitrile obtained in step (a) with hydrogen in the presence of at least one alkali metal hydroxide, water and a hydrogenation catalyst;
(c) separation of the alkali metal hydroxide by evaporation of the diamine obtained in step (b) and of the water;
(d) distillation of the diamine;
- the alkenenitrile is chosen from linear or branched C₂ to C₄ alkenes in which a hydrogen atom is replaced with a conjugated cyano group and
- the monoamine is a secondary amine of general formula R¹R²NH, in which R¹ and R², which may be identical or different, represent a C₁ to C₄ alkyl.

2. Process according to Claim 1, for which the monoamine is dimethylamine, the alkenenitrile is acrylonitrile, the aminonitrile is 3-(dimethylamino)propanenitrile and the diamine is dimethylaminopropylamine.

3. Process according to any one of Claims 1 to 3, for which step (a) is performed in one or more reactors in series of piston type with recycling of the excess monoamine and recycling of some of the aminonitrile.

4. Process according to any one of Claims 1 to 3, for which, in step (a), the monoamine is introduced in an excess of at least 0.1 mol% relative to the alkenenitrile.

5. Process according to any one of Claims 1 to 4, for which step (b) is performed in a reactor comprising means for separating out the catalyst.

6. Process according to any one of Claims 1 to 5, for which the catalyst of step (b) is separated out, and all or part of this catalyst is regenerated before being recycled into the reaction (b).

7. Process according to any one of Claims 1 to 6, for which the catalyst of step (b) is a Raney metal.

8. Process according to any one of Claims 1 to 7, for which
- the fraction of alkali metal hydroxide that is soluble in the reaction medium is removed by means of an evaporator;
- the water and the majority of the diamine derived from step (b) pass into vapor phase and are directed toward a distillation column for step (d).

9. Process according to Claim 8, for which the residual fraction of diamine associated with the alkali metal hydroxide leaving the evaporator is recovered by decantation after dissolution of the alkali metal hydroxide in water.

10. Process according to any one of Claims 1 to 9, for which, in step (c), the distillation is performed using a side-withdrawal column or a wall column.

11. Device for preparing diamine, comprising:
(a) at least a first reactor for the reaction between the corresponding monoamine and the corresponding alkenenitrile to form the corresponding aminonitrile, and a system for recycling the excess monoamine introduced;
(b) a second reactor having means for separating out the catalyst for the hydrogenation of the aminonitrile obtained, in the presence of an alkali metal hydroxide, a hydrogenation catalyst and water;
(c) an evaporator for separating out the alkali metal hydroxide that is soluble in the water + diamine mixture;
(d) a distillation column for purifying the diamine;
for which the recycling system consists of a separation system, a vacuum device and an absorption system;
for which the separation system makes it possible to recover:
- on the one hand, the aminonitrile, a first fraction of which is sent to the hydrogenation reactor and a second fraction of which is sent to an absorption system to enable dissolution of the excess monoamine; and
- on the other hand, the excess monoamine in gaseous form, which is sent, by means of a vacuum device, to the absorption system which enables its dissolution in the second fraction of aminonitrile, the whole then being sent to the first reactor.

12. Device according to Claim 11, for which the distillation column for purifying the diamine is a side-withdrawal column or a wall column.
